# EUROPEAN PATENT SPECIFICATION

(11) **EP 0 909 312 B1**
(45) Date of publication and mention of the grant of the patent: **04.12.2002**
(21) Application number: 97928141.7
(22) Date of filing: 04.07.1997
(51) Int. Cl.: C12N 1/19, C07K 14/605, C12N 15/81

(54) **METHOD FOR THE PRODUCTION OF POLYPEPTIDES**
VERFAHREN ZUR PRODUKTION VON POLYPEPTIDEN
PROCEDE DE PRODUCTION DE POLYPEPTIDES

(30) Priority: 05.07.1996 DK 74996
(43) Date of publication of application: 21.04.1999
(73) Proprietor: NOVO NORDISK A/S, 2880 Bagsvaerd (DK)
(72) Inventor: EGEL-MITANI, Michi, DK-2950 Vedbaek (DK); BRANDT, Jakob, DK-2700 Bronshoj (DK); VAD, Knud, DK-2000 Frederiksberg (DK)
(74) Representative: Secher, Anne
(86) International application number: DK9700298
(87) International publication number: WO98001535

(56) References cited:
- WO-A-95/23857
- DIALOG INFORMATION SERVICE, File 154, Medline, Dialog Accession No. 06480997, Medline Accession No. 90382674, GABRIELSEN O.S. et al., "Efficient Secretion of Human Parathyroid Hormone by Saccharomyces Cerevisiae"; & GENE, (NETHERLANDS), 15 Jun. 1990, 90(2), p. 255-62.
- DIALOG INFORMATION SERVICE, File 154, Medline, Dialog Accession No. 06460032, Medline Accession No. 90224362, EGEL-MITANI M. et al., "A Novel Aspartyl Protease Allowing KEX2-Independent MF Alpha Propheremone Processing in Yeast"; & YEAST, (ENGLAND), Mar.-Apr. 1990, 6(2), p. 127-37.

### SEQUENCE LISTING

### INFORMATION FOR SEQ ID NO:1

(i) SEQUENCE CHARACTERISTICS:
   (A) LENGTH: 41 amino acids
   (B) TYPE: amino acid
   (D) TOPOLOGY: linear
(ii) MOLECULE TYPE: peptide
(xi) SEQUENCE DESCRIPTION: SEQ ID NO:1:
   Gln Pro Ile Asp Asp Thr Glu Ser Asn Thr Thr Ser Val Asn Leu
   1               5                   10                  15
   Met Ala Asp Asp Thr Glu Ser Arg Phe Ala Thr Asn Thr Thr Leu
                   20                  25                  30
   Ala Leu Asp Val Val Asn Leu Ile Ser Met Ala
                   35                  40

### INFORMATION FOR SEQ ID NO:2

(i) SEQUENCE CHARACTERISTICS:
   (A) LENGTH: 52 amino acids
   (B) TYPE: amino acid
   (D) TOPOLOGY: linear
(ii) MOLECULE TYPE: peptide
(xi) SEQUENCE DESCRIPTION: SEQ ID NO:2:
   Glu Glu Ile Asp Ile Pro Ile Tyr Glu Lys Lys Tyr Gly Gln Val
   1               5                   10                  15
   Pro Met Cys Asp Ala Gly Glu Gln Cys Ala Val Arg Lys Gly Ala
                   20                  25                  30
   Arg Ile Gly Lys Leu Cys Asp Cys Pro Arg Gly Thr Ser Cys Asn
   35 40 45
   Ser Phe Leu Leu Lys Cys Leu
   50

### INFORMATION FOR SEQ ID NO:3

(i) SEQUENCE CHARACTERISTICS:
   (A) LENGTH: 41 amino acids
   (B) TYPE: amino acid
   (D) TOPOLOGY: linear
(ii) MOLECULE TYPE: peptide
(xi) SEQUENCE DESCRIPTION: SEQ ID NO:3:
   Ser Glu Glu Pro Pro Ile Ser Leu Asp Leu Thr Phe His Leu Leu
   1 5 10 15
   Arg Glu Val Leu Glu Met Ala Arg Ala Glu Gln Leu Ala Gln Gln
   20 25 30
   Ala His Ser Asn Arg Lys Leu Met Glu Ile Ile
   35 40

## Description

### FIELD OF THIS INVENTION

The present invention relates to a novel method for the production of short chain polypeptides, including polypeptides having up to 3 disulfide bonds and/or structures rich in basic amino acid residues, and open structured short chain polypeptides, e.g. glucagon, glucagon like peptides and their functional analogues, in genetically modified yeast cells, said genetically modified yeast cells, and a method for the preparation of said yeast cells.

### BACKGROUND OF THIS INVENTION

Expression of heterologous proteins in yeast after transformation of yeast cells with suitable expression vectors comprising DNA sequences coding for said proteins has been successful for many species of polypeptides, such as glucagon, glucagon like peptides and their functional analogues. Yeasts, and especially Saccharomyces cerevisiae, are preferred host microorganisms for the production of pharmaceutically valuable polypeptides due to the stable yield and safety.

However, it is often found that the expression product is a heterogeneous mixture of species of the desired polypeptide precursor having different amino acid chain lengths. A number of proteases, activated by the PEP4 gene product are responible for yeast protein degradation. Mutation in the PEP4 gene such as the pep4-3 mutation is often used to reduce cellular proteolysis whereby the quality and yields of heterologous proteins of interest can be improved. EP 341215 describes the use of a yeast strain that lacks carboxypeptidase yscα activity for the expression of the heterologous protein hirudin. Wild-type yeast strains produce a mixture of desulphatohirudin species differing in the C-terminal sequence due to the post-translational action of endogeneous yeast proteases on the primary expression product. It is shown that yeast mutant strains lacking carboxypeptidase yscα activity are unable to remove amino acids from the C-terminus of heterologous proteins and therefore give rise to integral proteins.
The use of yeast strains defective in protease A, B, Y, and/or S activity can only partially reduce random proteolysis of foreign gene products.

Another problem encountered in production of heterologous proteins in yeast is low yield, presumably due to proteolytic processing both in intracellular compartments and at the plasma membrane caused by aberrant processing at internal sites in the protein e.g. secretion of human parathyroid hormone (Gabrielsen et al. Gene 90: 255-262, 1990; Rokkones et al. J. Biotechnol. 33: 293-306, 1994), and secretion of β-endorphine by S. cerevisiae (Bitter et al. Proc. Natl. Acad. Sci. USA 81: 5330-5334, 1984). Some polypeptides, e.g. polypeptides having from about 10 to about 55 amino acids or shorter chains and none or a few disulphide bonds and/or are rich in basic amino acids, such as β-endorphine, glucagon and glucagon like peptides may be especially susceptible to intracellular and extracellular proteolytic degradation when expressed in a heterologous host due to their short-chain open and non-stable structure resulting in an inhomogeneous product.

WO 95/23857 discloses production of recombinant human albumin (rHA), which is a very large carrier-type protein cross-linked with 17 disulphide bonds and having a molecular weight of about 66 kD, in yeast cells having a reduced level of yeast aspartyl protease 3 (Yap3p) proteolytic activity resulting in a reduction of undesired 45 kD rHA fragment and in a 30 to 50% increased yield of recovered rHA produced by the haploid Δyap3 yeast strain compared to the rHA produced by the corresponding haploid YAP3 wild-type yeast strain.

Previously, Bourbonnais et al. (Biochimie 76: 226-233, 1994), have shown that the YAP3 protease gene product has in vitro substrate specificity which is distinct though overlapping with the Kex2p substrate specificity, and shown that Yap3p cleaves exclusively C-terminal to arginine residues present in the prosomatostatin's putative processing sites. Moreover, Cawley et al. (J. Biol. Chem. 271: 4168-4176, 1996) have determined the in vitro specificity and relative efficiency of cleavage of mono- and paired-basic residue processing sites by Yap3p for a number of prohormone substrates, such as bovine proinsulin.

The purpose of the present invention is to provide an improved method for the production of secreted polypeptides having up to about 55 amino acids, preferably from 10-50 amino acids, more preferably from 15-40 or preferably from 25-35 amino acids in the polypeptide chain, and having from 0 to 3 disulphide bonds, preferably no more than one disulphide bond, in the structure in a yeast expression system. Preferred examples of polypeptides are glucagon and glucagon like peptides, CRF, and truncated and/or C-or N-terminally truncated and/or N-terminally extended forms of cocaine amphetamine regulated transcript (CART). Preferably, the production of polypeptides according to the invention is considerably increased, e. g. more than two fold compared to the production of said polypeptides in conventional yeast expression systems.

Often it is advantageous to produce heterologous polypeptides in a diploid yeast culture, because possible genetical defects may become phenotypically expressed in a haploid yeast culture, e.g. during continuous fermentation in production scale, and because the yield may be higher (Fu et al. Biotechnol. Prog., 12: 145-148, 1996; Mead et al. Biotechnol. Letters, 8: 391-396, 1986).

It would be obvious for a person skilled in the art to use the method of the present invention to produce other polypeptides satisfying the above criteria, such as insulin and insulin analogues, adrenocorticotropic hormones, angiotensinogen, atrial natriuretic peptides, dynorphin, endorphines, galanin, gastrin, gastrin releasing peptides, neuropeptide Y fragments, pancreastatin, pancreatic polypeptides, secretin, vasoactiv intestinal peptide, growth hormone releasing factor, melanocyte stimulating hormone, neurotensin, adrenal peptide, parathyroid hormone and related peptides, somatostatin and related peptides, brain natriuretic peptide, calcitonin, corticotropin releasing factor (CRF), cf. SEQ ID NO: 3 herein, thymosin, and urotensin; and homologous or otherwise related peptides and fragments of these and other polypeptides (e.g. EEID-CART₅₅₋₁₀₂, cf. SEQ ID NO: 2 herein), as long as the criteria of having up to 55 amino acids, preferably from 10-50 amino acids, more preferably from 15-40 or from 25-35 amino acids in the polypeptide chain, and having from 0 to 3 disulphide bonds, preferably no more than one disulphide bond in the structure, is fulfilled.

### SUMMARY OF THE INVENTION

The above identified purpose is achieved with the method according to the present invention which comprises culturing a yeast which has reduced activity of Yap3 protease (Yap3p) or a homologue thereof and has been transformed with a hybrid vector comprising a yeast promoter operably linked to a DNA sequence coding for a polypeptide having up to 55 amino acids, preferably from 10-50 amino acids, preferably from 15-40, or from 25-35 amino acids in the polypeptide chain, and having from 0 to 3 disulphide bonds, preferably no more than one disulphide bond in the structure, such as glucagon or glucagon like peptides, and isolating said polypeptides. Preferably, the yeast cells lack Yap3p activity through disruption of the YAP3 gene.

Using a YAP3 disrupted yeast strain for the production of polypeptides having from 1-70 amino acids, preferably from 1-40, and more preferably from 10-30 amino acids in the polypeptide chain, and having no more than one disulphide bonds in the structure such as polypeptides encoded by the glucagon precursor gene including glucagon, GRPP, GLP-1, GLP-2, and their functional analogues thereof result in a remarkably improved yield of up to about 2-fold and even 10-fold compared to the yield from the corresponding YAP3 wild-type yeast strain. It has been found that using a YAP3 disrupted yeast strain for the production of heterologous polypeptides having up to 55 amino acids, preferably from 10-50 amino acids, preferably from 15-40 or from 25-35 amino acids, in the polypeptide chain, and having from 0 to 3 disulphide bonds, preferably no more than one disulphide bond in the structure, such as polypeptides encoded by the glucagon precursor gene including glucagon, GRPP, GLP-1, GLP-2, and their functional analogues thereof or CRF, e.g. as shown in SEQ ID NO:3 herein, or truncated and/or N-terminally extended forms of CART, preferably EEID-CART₅₅₋₁₀₂ as shown in SEQ ID NO:2 herein, result in a remarkably improved yield of the heterologous polypeptide of up to about 2-fold and even 10-fold compared to the yield obtained from the corresponding YAP3 wild-type yeast strain. Another advantage of using the method of the invention for production of heterologous polypeptides is that the secreted product has an improved homogenicity due to a reduced degree of proteolytic degradation.

The present inventors have also found that the use of a diploid YAP3 disrupted yeast in the method of the invention results in a significantly higher production level of secreted heterologous polypeptide which is about 2-fold and even 9-fold higher compared to the yield level from the corresponding wild-type haploid yeast.

Suitably, the yeast is S. cerevisiae which lacks a functional YAP3 gene. However, other yeast genera may have equivalent proteases, i.e. homologues of Yap3p, e. g. the genera Pichia and Kluyveromyces as shown in WO 95/23857 and Clerc et al. (J. Chromat. B. 662: 245-259, 1994). A gene is regarded as a homologue, in general, if the sequence of the translation product has greater than 50% sequence identity to Yap3p. Komano and Fuller (Proc. Natl. Acad. Sci, USA 92: 10752-10756, 1995) has identified the Mkc7 aspartyl protease from S. cerevisiae which is closely related to Yap3p (53% identity). Other aspartyl proteases of Saccharomyces include the gene products of PEP4, BAR1, and of open reading frames, the sequences of which are partially homologous with the YAP3 open reading frame, such as YAP3-link (coded by GenBank acc. No. X89514: pos. 25352-26878), YIV9 (Swiss Prot acc. No. P40583), and aspartyl protease (IV) (coded by GenBank acc. No. U28372: pos. 326-2116). According to recently accepted yeast genome nomenclature the yeast gene names YAP3, YAP3 link, YIV 9, NO 4, and MKC 7 used herein correspond to the yeast open reading frame YLR120C, YLR121C, YIR039C, YDR349C, and YDR144C, respectively. Furthermore, the gene product of open reading frame YGL259W is included among the yeast aspartyl proteases.

Examples of yeasts include Saccharomyces cerevisiae, Saccharomyces kluyveri, Schizosaccharomyces pombe, Kluyveromyces lactis, Hansenula polymorpha, Pichia pastoris, Pichia methanolica, Pichia kluyveri, Yarrowia lipolytica, Candida sp., Candida utilis, Candida cacaoi, Geotrichum sp., and Geotrichum fermentans.

A suitable means of eliminating the activity of a protease is to disrupt the host gene encoding the protease, thereby generating a non-reverting strain missing all or part of the gene for the protease including regulatory and/or coding regions, or, alternatively, the activity can be reduced or eliminated by classical mutagenesis procedures or by the introduction of specific point mutations. Other methods which may be suitable for down regulation of the protease include the introduction of antisense and/or ribozyme constructs in the yeast, e.g. Atkins et al. (Antisense and Development 5: 295-305, 1995) and Nasr et al. (Mol. Gen Genet 249: 51-57, 1995). One preferred method of disrupting the YAP3 gene in the yeast strain used in the method of the present invention are described by Rothstein (Method in Enzymol, 194: 281-301, 1991).

The expression "glucagon or glucagon like peptides" as used herein may be of human origin or from other animals and recombinant or semisynthetic sources and include all members of the glucagon family, such as GRPP (glicentine related polypeptide), glucagon, GLP-1 (glucagon like peptide 1), and GLP-2 (glucagon like peptide 2), including truncated and/or N-terminally extended forms, such as GLP-1(7-36), and includes analogues, such as GLP-1(7-35)R36A GLP-2 F22Y, GLP-2 A19T+34Y. GLP-2 A2G and GLP-2 A19T, and other analogues having from 1 to 3 amino acid changes, additions and/or deletions. The cDNA used for expression of the polypeptide according to the invention include codon optimised forms for expression in yeast.

Throughout the description and claims is used one and three letter codes for amino acids in accordance with the rules approved (1974) by the IUPAC-IUB Commission on Biochemical Nomenclature, vide Collected Tentative Rules & Recommendations of the Commission on Biochemical Nomenclature IUPAC-IUB, 2^{nd} ed., Maryland, 1975.

A further aspect of the invention is a culture of yeast cells transformed with a hybrid vector containing a polynucleotide sequence, preferably a DNA sequence, encoding a polypeptide having up to 55 amino acids, preferably from 10 to 50 amino acids, more preferably from 15 to 40, or preferably from 20 to 30 amino acids, most preferably from 25 to 35 amino acids in the polypeptide chain, and having from 0 to 3 disulphide bonds, preferably no more than one disulphide bond in the structure, said polynucleotide sequence or DNA sequence being operably linked to a polynucleotide sequence or DNA sequence encoding a yeast promoter and a leader sequence (pro sequence or prepro sequence) and/or other polynuceotide sequences or DNA sequences that are necessary for said polypeptide to be expressed in and secreted from the yeast, said culture of yeast cells being characterized in that the cells have reduced Yap3p activity, preferably through a disruption of the YAP3 gene, and said culture of yeast cells being a culture of haploid or polyploid, preferably diploid, yeast cells.

In another aspect the invention provides a culture of yeast cells containing a polynucleotide sequence encoding a polypeptide having up to 55 amino acids, preferably from 10-50 amino acids, preferably from 15-40 or preferably from 20-30 amino acids, most preferably from 25 to 35 amino acids, and having from 0 to 3 disulphide bonds, preferably one or less disulphide bonds in the structure, and a second polynucleotide sequence encoding a secretion signal causing said polypeptide to be expressed in and secreted from the yeast, characterized in that the yeast cells have reduced Yap3 protease activity. Preferably, the yeast cells are diploid yeast cells transformed with a hybrid vector comprising said polynucleotide sequences, and preferably the yeast cells lack Yap3p activity which may conveniently be obtained through disruption of the YAP3 gene.

The DNA encoding the polypeptides having up to 55 amino acids, preferably from 10-50 amino acids, preferably from 1540, or preferably from 25-35 amino acids in the polypeptide chain, and having from 0 to 3 disulphide bonds, preferably no more than one disulphide bonds in the structure, may be joined to a wide variety of other DNA sequences for introduction into an appropriate host. The companion DNA will depend upon the nature of the host, the manner of the introduction of the DNA into the host, and whether episomal maintenance or integration on host chromosome(s) is desired.

Generally, the DNA is inserted into an expression vector, such as a plasmid, in proper orientation and correct reading frame for expression. The vector is then introduced into the host through standard techniques and, generally, it will be necessary to select for transformed host cells.

If integration is desired, the DNA is inserted into an yeast integration plasmid vector, such as pJJ215, pJJ250, pJJ236, pJJ248, pJJ242 (Jones & Prakash, Yeast 6: 363,1990) or pDP6 (Fleig et al. Gene 46:237, 1986), in proper orientation and correct reading frame for expression, which is flanked with homologous sequences of any non-essential yeast genes, transposon sequence or ribosomal genes. Preferably the flanking sequences are yeast protease genes or genes used as a selective marker. The DNA is then integrated on host chromosome(s) by homologous recombination occured in the flanking sequences, by using standard techniques shown in Rothstein (Method in Enzymol, 194: 281-301, 1991) and Cregg et al. (Bio/Technol. 11:905-910, 1993).

Host cells that have been transformed by the recombinant DNA of the invention are then cultured for a sufficient time and under appropriate conditions known to those skilled in the art in view of the teachings disclosed herein to permit the expression and secretion of the polypeptides to be produced according to the method of the invention, preferred examples of polypeptides being glucagon, glucagon like peptides, CRF and EEID-CART₅₅₋₁₀₂, or their functional analogues, which can then be recovered, as is known.

Useful yeast plasmid vectors include the POT (Kjeldsen et al. Gene 170: 107-112, 1996) and YEp13, YEp24 (Rose and Broach, Methods in Enzymol. 185: 234-279, 1990), and pG plasmids (Schena et al. Methods in Enzymol. 194: 289-398, 1991).

Methods for the transformation of S. cerevisiae include the spheroplast transformation, lithium acetate transformation, and electroporation, cf. Methods in Enzymol. Vol. 194 (1991). Pereferably the transformation is as described in the examples herein.

Suitable promoters for S. cerevisiae include the MFα1 promoter, galactose inducible promoters such as GAL1, GAL7 and GAL10 promoters, glycolytic enzyme promoters including TPI and PGK promoters, TRP1 promoter, CYCI promoter, CUP1 promoter, PHO5 promoter, ADH1 promoter, and HSP promoter. A suitable promoter in the genus Pichia is the AOXI (methanol utilisation) promoter.

The transcription terminal signal is preferably the 3' flanking sequence of a eucaryotic gene which contains proper signal for transcription termination and polyadenylation. Suitable 3' flanking sequences may, e.g. be those of the gene naturally linked to the expression control sequence used, i.e. corresponding to the promoter.

The DNA constructs that are used for providing secretory expression of the polypeptide according to the invention comprise a DNA sequence that includes a leader sequence linked to the polypeptide by a yeast processing signal. The leader sequence contains a signal peptide ("pre-sequence") for protein translocation across the endoplasmic reticulum and optionally contains an additional sequence ("pro-sequence"), which may or may not be cleaved within yeast cells before the polypeptide is released into the surrounding medium. Useful leaders are the signal peptide of mouse α-amylase, S. cerevisiae MFα1, YAP3, BAR1, HSP150 and S. kluyveri MFα signal peptides and prepro-sequences of S. cerevisiae MFα1, YAP3, PRC, HSP150, and S. kluyveri MFα and synthetic leader sequences described in WO 92/11378, WO 90/10075 and WO 95/34666. Furthermore, the polypeptides to be produced according to the method of the invention may be provided with an N-terminal extension as described in WO 95/35384.

The invention also relates to a method of preparing a yeast having reduced Yap3p activity comprising the steps of a) providing a hybrid plasmid containing a part of the YAP3 gene and suitable for transformation into a yeast cell, b) disrupting the YAP3 gene by deleting the fragment of YAP3 and inserting the URA3 gene instead to obtain a Δyap3::URA3 gene disruption plasmid, c) providing a yeast Δura3 deletion mutant, d) transforming said mutant with said plasmid, and e) selecting the Δyap3::URA3 deletion mutants on a medium without uracil. Further the invention relates to a method of preparing a yeast having reduced Yap3p activity using antisense technology.

Moreover, the polypeptides to be produced according to the method of the invention may conveniently be expressed coupled to an N- or C-terminal tag or as a precursor or fusion protein although the total length of the expressed polypeptide may exceed a total of 55 or 70 amino acids.

### BRIEF DESCRIPTION OF THE DRAWINGS

Fig. 1 shows the construction of the pS194 plasmid.
Fig. 2 shows the construction of plasmids pME834 and pME1389.
Fig. 3 is a restriction map of the human glucagon expression plasmid pMT703.
Fig. 4 is a restriction map of the human GLP-1(7-37) expression plasmid pLaC253.
Fig. 5 is a restriction map of the human GLP-2 expression plasmid pKV210.
Fig. 6 is a restriction map of the pME973 plasmid, containing the genes encoding the HO (homothallism) endonuclease and Ura3p inserted into the YEp13 plasmid.

### DETAILED DESCRIPTION OF THIS INVENTION

Preferred embodiments of this invention are described in Table 1 below. Having knowledge of the art, it will be obvious to a skilled person to produce other polypeptides having up to 55 amino acids, preferably from 10-50 amino acids, preferably from 15-40, or preferably from 25-35 amino acids in the polypeptide chain, and having from 0 to 3 disulphide bonds, preferably no more than one disulphide bond in the structure and their functional analogues by the method of the present invention using similar constructs.

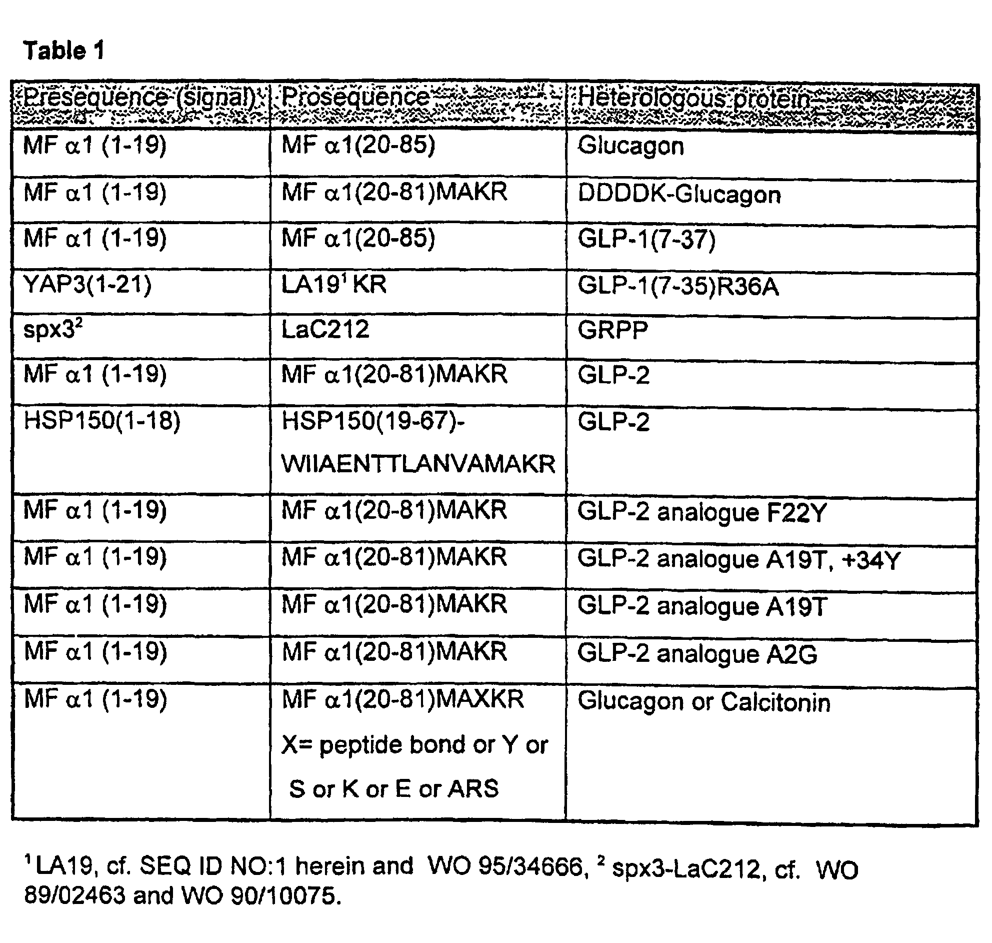

The Genetic background of S. cerevisiae strains used herein is as follows:

| | |
|---|---|
| E11-3C | MATα YAP3 pep4-3 Δtpi::LEU2 leu2 URA3 |
| SY107 | MATα YAP3 pep4-3 Δtpi::LEU2 leu2 Δura3 |
| ME1487 | MATα Δyap3::URA3 pep4-3 Δtpi::LEU2 leu2 Δura3 |
| ME1656 | MATα Δyap3::ura3 pep4-3 Δtpi::LEU2 leu2 Δura3 |
| ME1684 | MAT**a** Δyap3::URA3::Δylr121c pep4-3 Δtpi::LEU2 leu2 Δura3 |
| ME1695 | MATα Δyap3::ura3 pep4-3 Δtpi::LEU2 leu2 Δura3 |
| ME1719 | MAT**a**/α Δyap3::URA3/Δyap3::ura3 pep4-3/pep4-3 Δtpi::LEU2/Atpi::LEU2 leu2/leu2 Δura3/Δura3 |
| MT663 | MAT**a**/α YAP3/YAP3 pep4-3/pep4-3 Δtpi::LEU2/Δtpi::LEU2 leu2/leu2 URA3/URA3 HIS4/his4 |

The present invention is further illustrated by the following examples which, however, are not to be construed as limiting the scope of protection. The features disclosed in the fore-going description and in the following examples may, both separately and in any combination thereof, be material for realizing the invention in diverse forms thereof.

### Example 1

### Δyap3::URA3 gene disruption

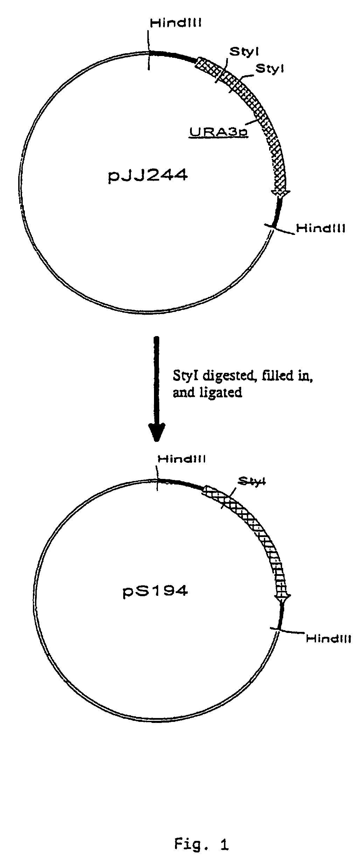

The Δura3 deletion mutation was constructed as follows:
pJJ244 (pUC18 containing a 1.2 kb HindIII fragment of the URA3 gene) was digested with Styl and filled in with Klenow polymerase and self ligated. The resulting plasmid designated pS194 contains a 84bp of Styl-Styl fragment deletion of the URA3 gene, cf. Fig.1.

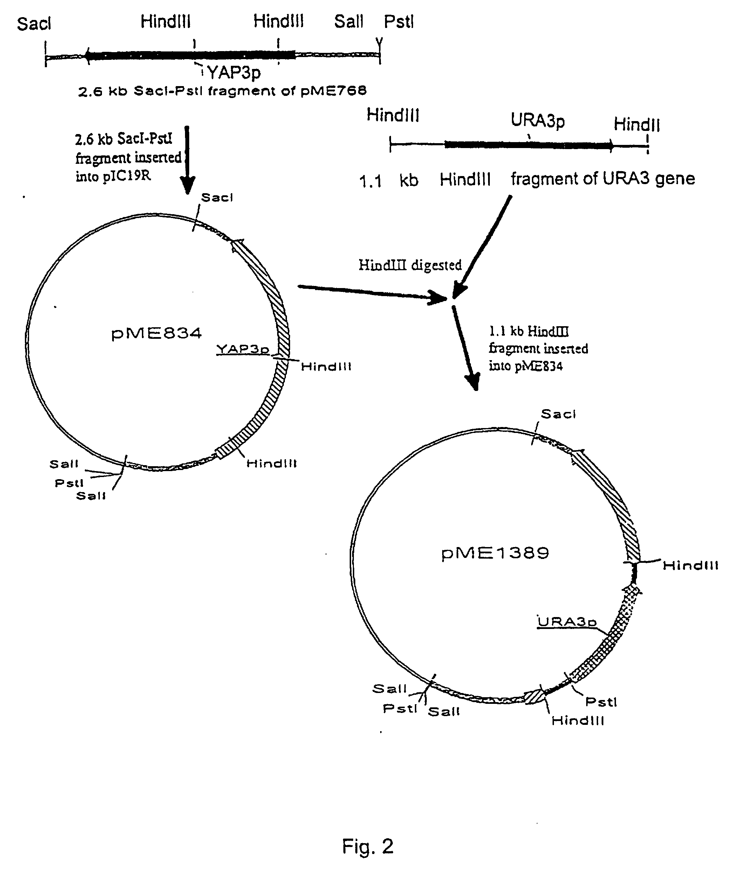

The Δyap3::URA3 gene disruption plasmid pME1389 was constructed as follows:
The 2.6kb SacI-PstI fragment which contains the YAP3 gene in pME768 (Egel-Mitani et al. Yeast 6: 127-137, 1990) was inserted in 2.6 kb of the Sacl-Pstl fragment of pIC19R (Marsh et al. Gene 32: 481-485, 1984). The resulting plasmid is pME834. pME834 was digested with HindIII to form a deletion of the 0.7 kb YAP3 fragment and the 1.2 kb HindIII fragment of the URA3 gene (Rose et al. Gene 29: 113-124, 1984) was inserted instead. The resulting plasmid is pME1389. The construction of plasmids pME834 and pME1389 is shown in Fig. 2 in diagrammatic form.

S. cerevisiae strain E11-3C (MATα YAP3 pep4-3 Δtpi::LEU2 leu2 URA3), cf. ATCC 20727, US pat. 4766073, was transformed with linialized pS194 (Bsgl digested) to make Δura deletion mutation. By selection on 5-FOA (5-fluoro-orotic acid) containing minimal plates, the Δura3 mutant designated Sy107 was obtained.

The strain Sy107 (MATα YAP3 pep4-3 Δtpi::LEU2 leu2 Δura3). was then transformed with pME1389 previously being cut by SalI and SacI, and 3kb fragment of Δyap3::URA3 was isolated for the transformation. Δyap3::URA3 deletion mutants were selected on minimal plates without uracil. URA3 transformants were characterized by PCR and Southern hybridisation to confirm the correct integration of the Δyap3::URA3 fragment in the YAP3 locus. ME1487 was isolated as a Δyap3::URA3 deletion mutant (MATα Δyap3::URA3 pep4-3 Δtpi::LEU2 leu2 Δura3).

### Example 2

### Construction of a diploid Δyap3/Δyap3 strain

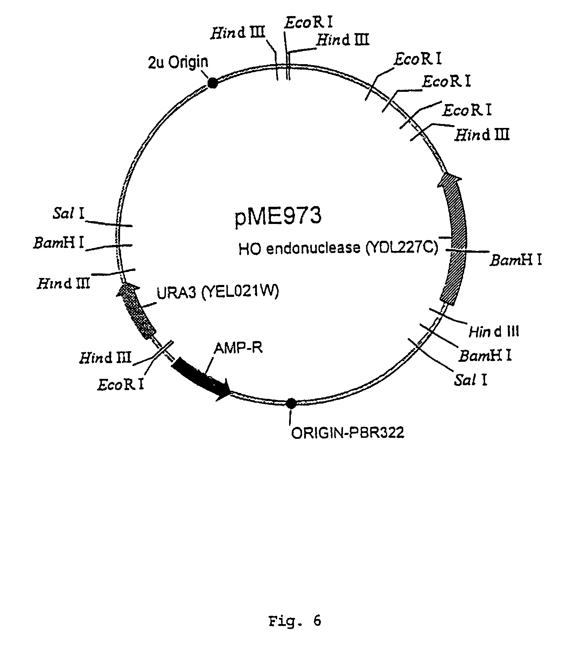

ME1487 was mutagenized by using EMS (methane-sulfonic acid ethylester) and ura3 mutants were selected on plates containing 5-FOA. One of the selected isolates, ME1656 was then subjected to mating type switch (Herskowitz and Jensen, Methods in Enzymol. 194: 132-146, 1991) by transient transformation with pME973 shown in Fig. 6. pME973 contains the genes encoding the HO (homothallism) endonuclease and URA3 inserted into the YEp13 plasmid (Rose and Broach, Methods in Enzymol. 185: 234-279, 1990). From transient transformants, ME1695 was selected as the haploid strain, which had switched from MATα to MAT**a**, and have the following genetic background: MAT**a** Δyap3::ura3 pep4-3 Δtpi::LEU2 leu2 Δura3.

ME1695 was then crossed with ME1487 by micromanipulation (Sherman and Hicks, Methods in Enzymol. 194: 21-37, 1991) in order to get Δyap3/Δyap3 diploids. From the resulting diploids, ME1719 was selected as the strain with the following genetic background:
MAT**a**/α Δyap3::ura3/Δyap3::URA3 pep4-3/pep4-3 Δtpi::LEU2/Δtpi::LEU2 leu2/leu2 Δura3/Δura.

### Example 3

### Construction of a Δyap3::URA3::Δylr121c double disruption strain

In order to make a one-step gene disruption strain of the two closely linked genes encoding YAP3 and YLR121C, the following two oligonucleotide primers were synthesized:
- P1: Length 57bp: YLR121C/URA3 primer
- P2: Length 57bp: YAP3/URA3 primer
P1 and P2 each contains 40 nucleotides corresponding to sequences within the coding region of YLR121C and YAP3, respectively, as well as a HindIII site (AAGCTT) and 12 nucleotides corresponding to sequences flanking the URA3 gene (YEL021W). P1 and P2 were used for PCR using the URA3 gene as template. The resulting 1248bp PCR fragment contains the URA3 selective marker flanked with 40 nucleotides derived from the YAP3 or YLR121C encoding regions. The PCR fragment was then transformed into ME1655, and Δyap3::URA3::Δylr121c deletion mutants were selected and characterized as described in Example 1. ME1684 was isolated as a Δyap3::URA3::Δylr121c mutant with the following genetic background: MATα Δyap3::URA3::Δylr121c pep4-3 Δtpi::LEU2 leu2 Δura3.

### Example 4

### Transformation into yeast

In order to make yeast competent cells, yeast haploid strains SY107 and ME1487 or the diploid ME1719 strain were cultivated in 100ml YPGGE medium (1% yeast extract, 2% peptone, 2% glycerol, 2% galactose, 1% ethanol) to OD₆₀₀ = 0.2. Cells were harvested by centrifugation at 2000rpm for 5 min. and washed once by 10ml H₂O. Cells were resuspended in 10ml SED (1M sorbitol, 25mM Na₂EDTA pH8, 6.7mg/ml dithiothreitol) and incubated at 30°C for 15min. Cells were harvested by centrifugation and resuspended in 10ml SCE (1M sorbitol, 0.1M Na-citrate, 10mM Na₂EDTA, pH5.8) + 2mg Novozyme SP234 and incubated at 30°C for 30 min. After cells were harvested by centrifugation and washed once by 10ml 1.2M sorbitol and subsequently by 10ml CAS (1M sorbitol 10mM CaCl₂, 10mM Tris-HCI pH7.5), cells were harvested by centrifugation and resuspended finally in 2ml CAS. Competent cells were frozen in portion of 100µl per Eppendorf tube at -80°C.

Transformation was made as follows: Frozen competent cells (100µl) were warmed up quickly and 1µg plasmid DNA were added. Cells were incubated at room temp. for 15 min. and 1ml PEG solution (20% polyethyleneglycol 4000, 10mM CaCl₂, 10mM Tris-HCl pH7.5) was added. After 30min. at room temperature, cells were harvested by centrifugation at 2000rpm for 15min. and resuspended in 100µl SOS (1M sorbitol, 1/2 vol. YPGGE, 0.01% uracil, 7mM CaCl₂). After incubating at 30°C for 2 hours, cells were centrifuged and resuspended in 0.5ml 1M sorbitol. Cells were then spread on YPD plates (1% yeast extract, 2% peptone, 2% glucose, 2% agar) together with 6ml of top agar (YPD containing 2.5% agar). Plates were incubated at 30°C for 3 to 5 days until transformants appear.

### Example 5

### Heterologous protein expression plasmid

Yeast-E.coli shuttle vector used in the following examples contains a heterologous protein expression cassette, which includes a DNA sequence encoding a leader sequence followed by the heterologous polypeptide in question operably placed in between the TPI promoter and TPI terminator of S. cerevisiae in a POT plasmid (Kjeldsen et al. 1996, *op. cit*.). The leader sequences are the MFα1 prepro-sequence and modification thereof. Examples are shown as follows:

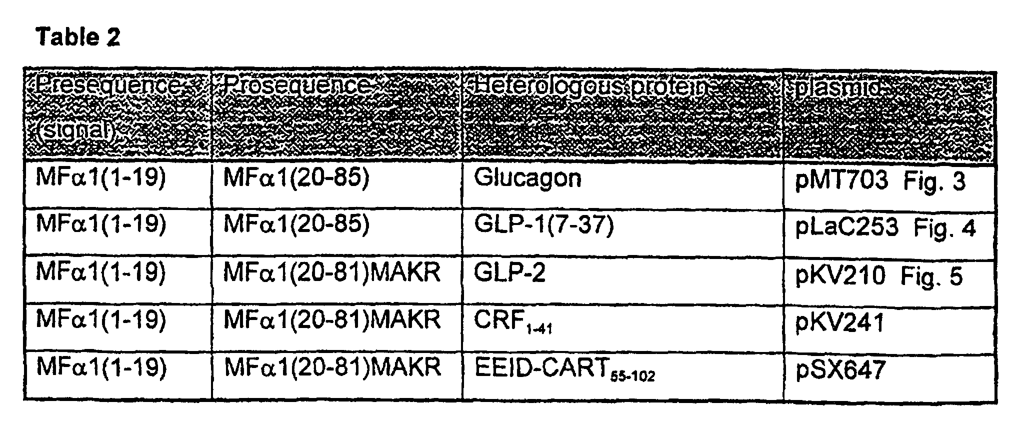

### Example 6

### Expression of glucagon

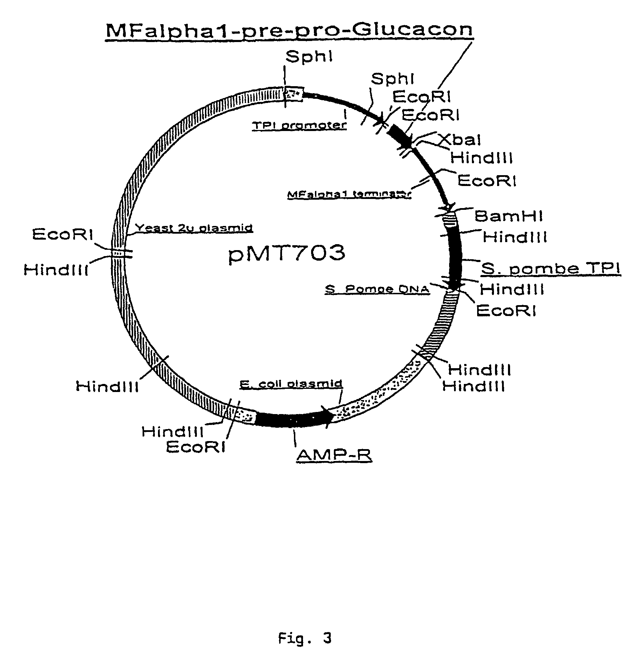

Human glucagon expression plasmid pMT703, cf. Figure 3, was transformed into three strains, such as, YAP3 disrupted haploid strain ME1487(Δyap3), YAP3 disrupted diploid strain ME1719 (Δyap3/Δyap3) and YAP3 wild-type strain SY107 (YAP3 WT). Transformants were selected by glucose utilization as a carbon source in YPD plates (1% w/v yeast extract, 2% w/v peptone, 2% glucose, 2% agar). ME1532 and YES1746 are pMT703 transformants obtained from ME1487 (Δyap3) and ME1719 (Δyap3/Δyap3), respectively, whereas ME1530 is the pMT703 transformant obtained from SY107 (YAP3 WT). Transformants were cultivated in 5ml YPD liquid medium at 30°C for 3 days with shaking at 200rpm. Culture supernatants were obtained after centrifugation at 2500rpm for 5 min. and 1ml supernatants were analyzed by reverse phase HPLC. Production levels, shown in Table 3, were average value of cultures from 2 independently isolated transformants (Exp.1) or values from a mixculture of 3 transformants (Exp.2), and were normalised so that the haploid YAP3 wild-type level was taken as 100%. HPLC analyses showed that ME1532 or YES1746 produced approx. 4 to 6 times more glucagon than ME1530.

### HPLC setting for glucacon detection

- HPLC-Column:: 4 x 250 mm Novo Nordisk YMC-OdDMeSi C18 5 µm
- Column temp.:: 50°C
- Flowrate:: 1 ml/min
- HPLC solvents:: A: 10 % (v/v) acetonitrile in 0.2 M Na₂SO₄, 0.04 M H₃PO₄ pH adjusted to 2.3 with ethanolamine
B: 50 % (v/v) acetonitrile in water
Inj. vol: 150 µl

Glucagon was eluated from the HPLC columns with 23.6 % acetonitrile to 32.9 % acetonitrile in 40 min.

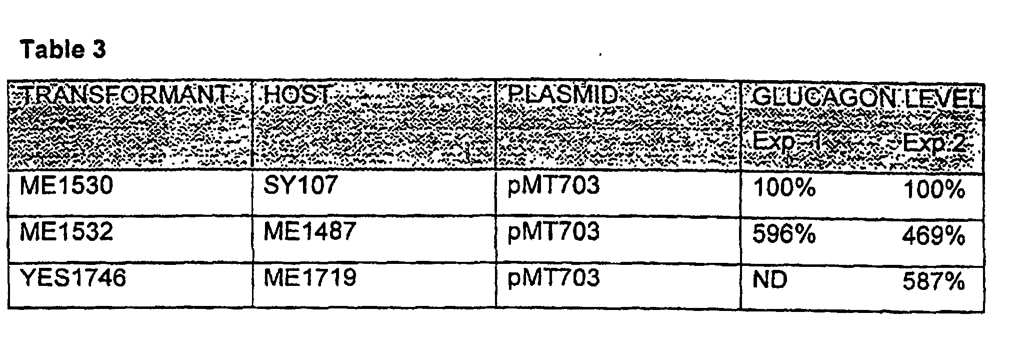

### Example 7

### Expression of GLP-1(7-37)

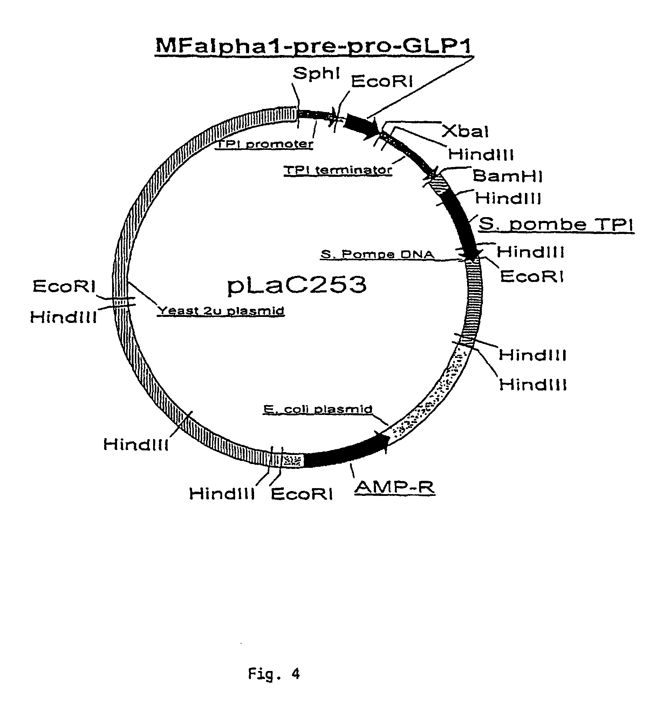

Human GLP-1(7-37) expression plasmid pLaC253, cf. Figure 4, was transformed into ME1487(Δyap3), ME1719 (Δyap3/Δyap3) and SY107 (YAP3 WT). Transformants were selected and analysed as described in Example 6. ME1535 and YES1823 are the pLaC253 transformants obtained from ME1487(Δyap3) and ME1719 (Δyap3/Δyap3), respectively, whereas ME1534 is the pLaC253 transformant obtained from SY107 (YAP3 WT). Production levels, shown in Table 4, were average value of cultures from 2 independently isolated transformants (Exp.1) or values from a mixculture of 3 transformants (Exp.2), and were normalised so that the haploid YAP3 wild-type level was taken as 100%. HPLC analyses showed that ME1535 or YES1823 produced approx. 2 to 3 times more GLP-1(7-37) than ME1530.

### HPLC settings for GLP-1(7-37) detection:

As described in Example 6, except that GLP-1 was eluated from HPLC columns with 31.2% acetonitrile to 41.2% acetonitrile in 40 min.

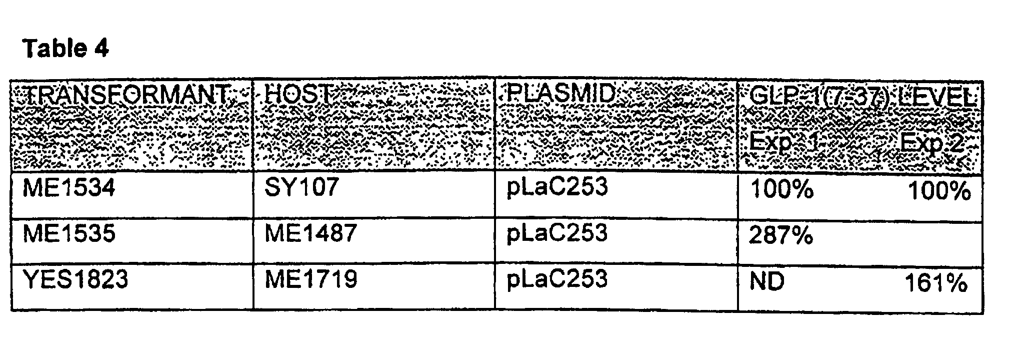

### Example 8

### Expression of GLP-2

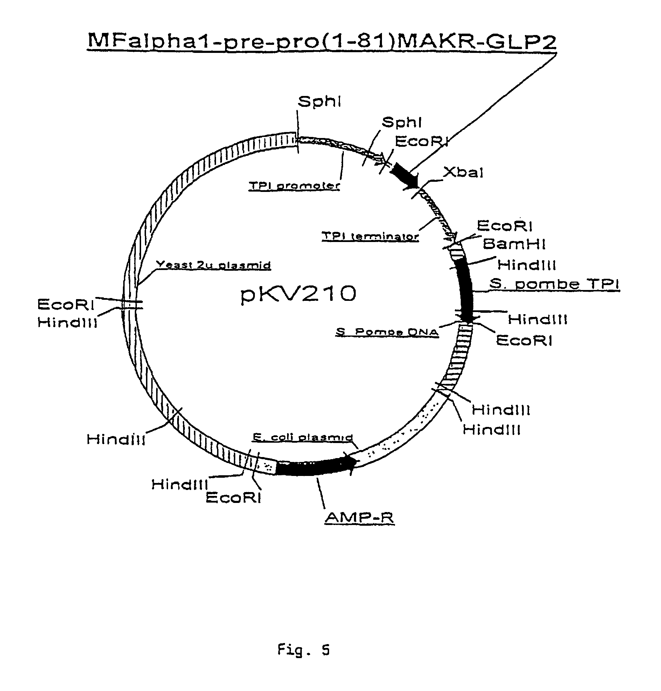

Human GLP-2 expression plasmid pKV210, cf. Figure 5, was transformed into ME1487 (Δyap3), ME1719 (Δyap3/Δyap3) and SY107 (YAP3 WT). Transformants were selected and analysed as described in Example 6. ME1615 and YES1827 are the pKV210 transformants obtained from ME1487 (Δyap3) and ME1719 (Δyap3/Δyap3), respectively, whereas ME1614 is the pKV210 transformant obtained from Sy107 (YAP3 WT). Production levels, shown in Table 5 were average value of cultures from 2 independently isolated transformants (Exp.1) or values from a mixculture of 3 transformants (Exp.2), and were normalized so that the YAP3 wild-type level was taken as 100%. HPLC analyses showed that ME1615 and YES1827 produced approx. 6 to11 times more GLP-2 than ME1614.

### HPLC settings for GLP-2 detection:

- HPLC-Column:: Vydac 214TP54 Column
- Flowrate:: 1 ml/min
- HPLC solvents:: A: 0.1% TFA
B: 0.07% TFA in acetonitrile

GLP-2 was eluated from the HPLC columns with 0.07% TFA in 20% to 80% acetonitrile in 60 min.

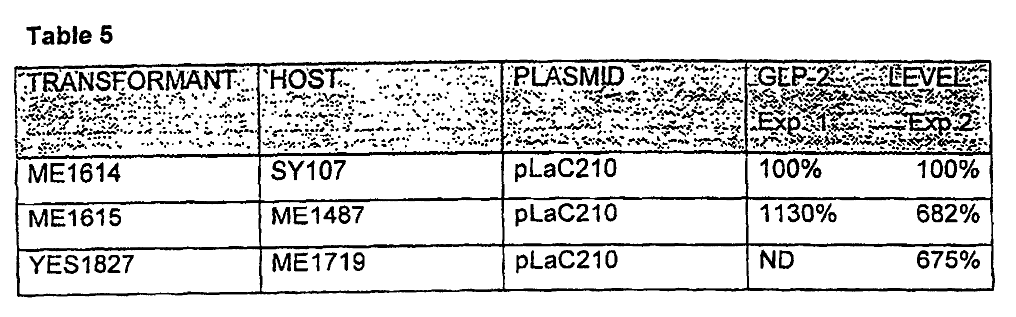

### Example 9

### Expression of CRF₁₋₄₁ (SEQ ID NO:3 herein)

Human Corticotropin Releasing Factor (CRF₁₋₄₁) expression plasmid pKV241 (equivalent to pKV210 Fig. 5 in which MFα1-pre-pro(1-81)MAKR-GLP2 is substituded by MFα1-pre-pro(1-81)MAKR-CRF₁₋₄₁) was transformed into ME1487(Δyap3), ME1719 (Δyap3/Δyap3) and SY107 (YAP3 WT). Transformants were selected and analysed as desribed in Example 6. ME1813 and YES1810 are the pKV241 transformants obtained from ME1487(Δyap3) and ME1719 (Δyap3/Δyap3), respectively, whereas ME1812 is the pKV241 transformant obtained from SY107 (YAP3 WT). Production levels, shown in Table 6, were values from a mixculture of 3 transformants (Exp.2), and were normalised so that the haploid YAP3 wild-type level was taken as 100%. HPLC analyses were performed as in example 8 and showed that ME1813 or YES1810 produced approx. 8 to 9 times more CRF₁₋₄₁ than ME1812.

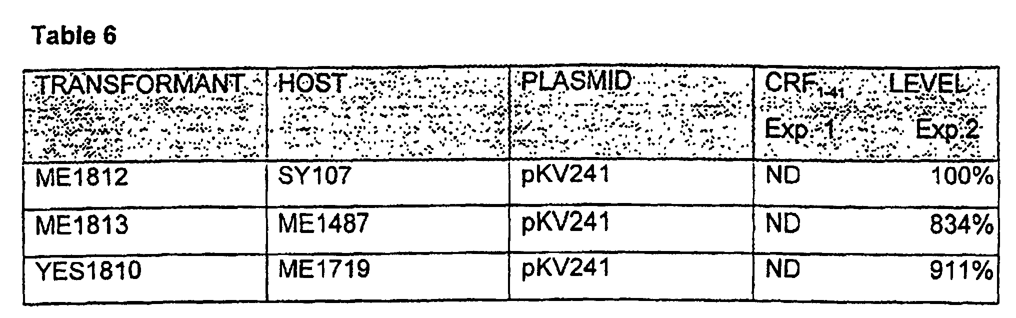

### Example 10

### Expression of EEID-CART₅₅₋₁₀₂(SEQ ID NO:2 herein)

N-terminal extended (EEID) fragment of Human Cocaine and Amphetamine regulated transcript (EEID-CART₅₅₋₁₀₂) expression plasmid pSX637 (equivalent to pKV210 Fig. 5, in which MFα1-pre-pro(1-81)MAKR-GLP2 is substituded by MFα1-pre-pro(1-81)MAKR-EEID-CART₅₅₋₁₀₂), was transformed into ME1487(Δyap3), ME1719 (Δyap3/Δyap3) and SY107 (YAP3 WT). Transformants were selected and analysed as described in Example 6. ME1817 and YES1820 are the pSX637 transformants obtained from ME1487(Δyap3) and ME1719 (Δyap3/Δyap3), respectively, whereas ME1816 is the pSX637 transformant obtained from SY107 (YAP3 WT). Production levels, shown in Table 7, were values from a mixculture of 3 transformants (Exp.2), and were normalised so that the haploid YAP3 wild-type level was taken as 100%. HPLC analyses were performed as in example 8 and showed that ME1817 or YES1820 produced approx. 2 to 3 times more EEID-CART₅₅₋₁₀₂ than ME1816.

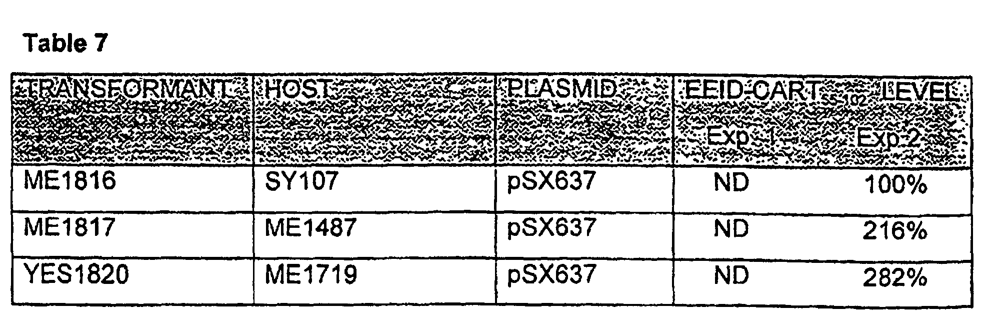

### Example 11

Table 8 shows data for expression levels of human glucagon, GLP-1₍₁₋₃₇₎ and GLP-2 in ME1487 (Δyap3) transformed by expression plasmids with different pre-pro-sequences (leaders). Expression plasmids are as descibed in Fig. 5 except for the details given in Table 8. Expression yields are normalised so that the yield obtained in MT663 (YAP3/YAP3) transformants is set to 100%.

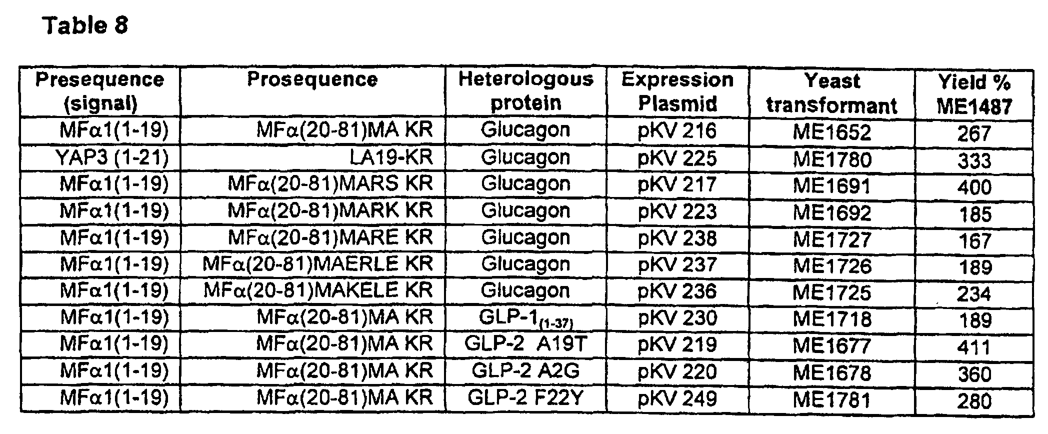

## Claims

1. A method for the production of a heterologous open structured short chain polypeptide in yeast comprising (a) culturing a yeast having reduced activity of Yap3 protease, said yeast being transformed with a hybrid vector comprising a yeast promoter operably linked to a DNA sequence coding for the heterologous polypeptide having from 10 to 70 amino acids in the polypeptide chain, and having from 0 to 3 disulphide bonds in the structure, and (b) isolating said polypeptide.

2. A method according to claim 1, wherein the heterologous polypeptide has from 10-55 amino acids in the polypeptide chain.

3. A method according to claim 1, wherein the heterologous polypeptide has from 10-50 amino acids in the polypeptide chain.

4. A method according to claim 1, wherein the heterologous polypeptide has from 15-40 amino acids in the polypeptide chain.

5. A method according to claim 1, wherein the heterologous polypeptide has from 25-35 amino acids in the polypeptide chain.

6. A method according to any of claims 1 to 5, wherein the heterologous polypeptide has one disulphide bond in the structure.

7. A method according to any of claims 1 to 6, wherein the yeast lacks Yap3 protease activity.

8. A method according to claim 1, wherein the yeast is selected from the group consisting of Saccharomyces cerevisiae, Saccharomyces kluyveri, Schizosaccharomyces pombe, Kluyveromyces lactis, Hansenula polymorpha, Pichia pastoris, Pichia methanolica, Pichia kluyveri, Yarrowia lipolytica, Candida sp., Candida utilis, Candida cacaoi, Geotrichum sp., and Geotrichum fermentans.

9. A method according to claim 8, wherein the yeast is S. cerevisiae.

10. A method according to any of claims 1 to 9, wherein the yeast additionally has reduced protease activity selected from the group of proteases coded for by BAR1, STE13, PRA, PRB, KEX1, PRC, CPS, and the YAP3 homologues MKC7, YAP3-link (coded by GenBank acc. No. X89514: pos.25352-26878), YIV9 (Swiss Prot acc. No. P40583), and aspartyl protease (IV) (coded by GenBank acc. No. U28372: pos. 326-2116) genes.

11. A method according to any of claims 1 to 9, wherein the yeast additionally has the pep4-3 mutation.

12. A method according to claim 1, wherein the yeast promoter is selected from the group consisting of the MFα1 promoter, the CYC1 promoter; the galactose inducible promoters GAL1, GAL7 and GAL10; the glycolytic enzyme promoters TPI and PGK; the TRP1 promoter, the CUP1 promoter; the PHO5 promoter; the ADH1 promoter; and the HSP promoter.

13. A method according to any one of the preceding claims, wherein the hybrid vector comprises a DNA sequence encoding a leader sequence being either a pre-sequence (signal) or a prepro-sequence linked in the proper reading frame to the DNA sequence coding for the heterologous polypeptide.

14. A method according to any one of the preceding claims, wherein the heterologous polypeptide is selected from the group consisting of GRPP (glicentine related polypeptide), glucagon, GLP-1 (glucagon like peptide 1), and GLP-2 (glucagon like peptide 2), GLP-1 (7-36), GLP-1(7-35)-(R36A), GLP-2-(F22Y), GLP-2-( A19T+34Y), GLP-2-(A2G) and GLP-2-(A19T), and other analogues having from 1 to 3 amino acid changes, additions and/or deletions.

15. A culture of yeast cells **characterized in that** they have reduced Yap3p activity and that they contain a first DNA sequence encoding a heterologous polypeptide having from 10-70 amino acids and having from 0 to 3 disulphide bonds in the structure, and a second DNA sequence encoding a leader sequence causing said polypeptide to be secreted from the yeast.

16. A culture according to claim 15, wherein the yeast cells lacks Yap3p activity.

17. A culture according to any one of claims 15 or 16, wherein the yeast is selected from the group consisting of Saccharomyces cerevisiae, Saccharomyces kluyveri, Schizosaccharomyces pombe, Kluyveromyces lactis, Hansenula polymorpha, Pichia pastoris, Pichia methanolica, Pichia kluyveri, Yarrowia lipolytica, Candida sp., Candida utilis, Candida cacaoi, Geotrichum sp., and Geotrichum fermentans.

18. A culture according to claim 17, wherein the yeast is S. cerevisiae.

19. A culture according to any one of the claims 15 to 18, wherein the yeast cells additionally have reduced protease activity selected from the group of proteases coded for by the BAR1, STE13, PRA, PRB, KEX1, PRC, CPS, and the YAP3 homologues MKC7, YAP3-link (coded by GenBank acc. No.X89514: pos. 25352-26878), YIV9 (Swiss Prot acc. No. P40583), and aspartyl protease (IV) (coded by GenBank acc. No. U28372: pos. 326-2116) genes.

20. A culture according to any one of claims 15 to 18, wherein the yeast cells additionally have the pep4-3 mutation.

21. A culture according to any one of claims 15 to 20, wherein the hybrid vector comprises a yeast promoter selected from the group consisting of the MFα1 promoter; CYC1 promoter; the galactose inducible promoters GAL1, GAL7 and GAL10; the glycolytic enzyme promoters TPI and PGK; the TRP1 promoter; the CUP1 promoter; the PHO5 promoter; the ADH1 promoter; and the HSP promoter.

## Patentansprüche

1. Verfahren zur Herstellung eines heterologen offen strukturierten kurzkettigen Polypeptids in Hefe umfassend (a) Kultivieren einer Hefe, die eine reduzierte Aktivität der Yap3 Protease aufweist, wobei die Hefe mit einem Hybridvektor transformiert ist, umfassend einen Hefepromotor, der operativ mit einer DNA-Sequenz verbunden ist, kodierend für das heterologe Polypeptid mit 10 bis 70 Aminosäuren in der Polypeptidkette, und das 0 bis 3 Disulfidbrücken in der Struktur aufweist, und (b) Isolieren des Polypeptids.

2. Verfahren nach Anspruch 1, wobei das heterologe Polypeptid von 10 - 55 Aminosäuren in der Polypeptidkette aufweist.

3. Verfahren nach Anspruch 1, wobei das heterologe Polypeptid von 10 - 50 Aminosäuren in der Polypeptidkette aufweist.

4. Verfahren nach Anspruch 1, wobei das heterologe Polypeptid von 15 - 40 Aminosäuren in der Polypeptidkette aufweist.

5. Verfahren nach Anspruch 1, wobei das heterologe Polypeptid von 25 - 35 Aminosäuren in der Polypeptidkette aufweist.

6. Verfahren nach einem der Ansprüche 1 bis 5, wobei das heterologe Polypeptid eine Disulfidbrücke in der Struktur aufweist.

7. Verfahren nach einem der Ansprüche 1 bis 6, wobei die Hefe keine Yap3 Proteaseaktivität aufweist.

8. Verfahren nach Anspruch 1, wobei die Hefe ausgewählt ist aus der Gruppe bestehend aus Saccharomyces cerevisiae, Saccharomyces kluyveri, Schizosaccharomyces pombe, Kluyveromyces lactis, Hansenula polymorpha, Pichia pastoris, Pichia methanolica, Pichia kluyveri, Yarrowia lipolytica, Candida sp., Candida utilis, Candida cacaoi, Geotrichum sp. und Geotrichum fermentans.

9. Verfähren nach Anspruch 8, wobei die Hefe S. cerevisiae ist.

10. Verfahren nach einem der Ansprüche 1 bis 9, wobei die Hefe zusätzlich eine reduzierte Proteaseaktivität aufweist, die ausgewählt ist aus der Gruppe von Proteasen, die kodiert werden durch BAR1, STE13, PRA, PRB, KEX1, PRC, CPS und den YAP3 homologen Genen MKC7, YAP3-link (kodiert durch GenBank Zugangs-Nr. X89514: Pos. 25352-26878), YIV9 (Swiss Prot Zugangs-Nr. P40583) und Aspartylprotease (IV) (kodiert durch GenBank Zugangs-Nr. U28372: Pos. 326-2116).

11. Verfahren nach einem der Ansprüche 1 bis 9, wobei die Hefe zusätzlich eine pep4-3 Mutation aufweist.

12. Verfahren nach Anspruch 1, wobei der Hefepromotor ausgewählt ist aus der Gruppe bestehend aus dem MFα1 Promotor; dem CYC1 Promotor; den galactoseinduzierbaren Promotoren GAL1, GAL7 und GAL10; den glycolytischen Enzympromotoren TPI und PGK; dem TRP1 Promotor, dem CUP1 Promotor, dem PHO5 Promotor, dem ADH1 Promotor und dem HSP Promotor.

13. Verfahren nach einem der vorhergehenden Ansprüche, wobei der Hybridvektor eine DNA-Sequenz umfasst, die eine Leadersequenz kodiert, die entweder eine Präsequenz (Signal-) oder eine Präprosequenz ist, die in dem richtigen Leserahmen mit der DNA-Sequenz kodierend für das heterologe Polypeptid verbunden ist.

14. Verfahren nach einem der vorhergehenden Ansprüche, wobei das heterologe Polypeptid ausgewählt ist aus der Gruppe bestehend aus GRPP (Glicentin verwandtes Polypeptid), Glucagon, GLP-1 (Glucagon ähnliches Peptid 1) und GLP-2 (Glucagon ähnliches Peptid 2), GLP-1(7-36), GLP-1(7-35)-(R36A), GLP-2-(F22Y), GLP-2-(A19T+34Y), GLP-2-(A2G) und GLP-2-(A19T) und andere Analoge, die 1 bis 3 Aminosäureveränderungen, Additionen und/oder Deletionen aufweisen.

15. Kultur von Hefezellen, **dadurch gekennzeichnet, dass** sie eine reduzierte Yap3p Aktivität aufweisen, und dass sie eine erste DNA-Sequenz enthalten, kodierend ein heterologes Polypeptid mit 10 - 70 Aminosäuren und mit 0 bis 3 Disulfidbrücken in der Struktur, und eine zweite DNA-Sequenz kodierend eine Leadersequenz, die dazu führt, dass das Polypeptid aus der Hefe sekretiert wird.

16. Kultur nach Anspruch 15, wobei die Hefezellen keine Yap3p Aktivität aufweisen.

17. Kultur nach einem der Ansprüche 15 oder 16, wobei die Hefe ausgewählt ist aus der Gruppe bestehend aus Saccharomyces cerevisiae, Saccharomyces kluyveri, Schizosaccharomyces pombe, Kluyveromyces lactis, Hansenula polymorpha, Pichia pastoris, Pichia methanolica, Pichia kluyveri, Yarrowia lipolytica, Candida sp., Candida utilis, Candida cacaoi, Geotrichum sp. und Geotrichum fermentans.

18. Kultur nach Anspruch 17, wobei die Hefe S. cerevisiae ist.

19. Kultur nach einem der Ansprüche 15 bis 18, wobei die Hefezellen zusätzlich eine reduzierte Proteaseaktivität aufweisen, die ausgewählt ist aus der Gruppe von Proteasen, die kodiert werden durch BAR1, STE13, PRA, PRB, KEX1, PRC, CPS und den YAP3 homologen Genen MKC7, YAP3-link (kodiert durch GenBank Zugangs-Nr. X89514: Pos. 25352-26878), YIV9 (Swiss Prot Zugangs-Nr. P40583) und Aspartylprotease (IV) (kodiert durch GenBank Zugangs-Nr. U28372: Pos. 326-2116).

20. Kultur nach einem der Ansprüche 15 bis 18, wobei die Hefezellen zusätzlich eine pep4-3 Mutation aufweisen.

21. Kultur nach einem der Ansprüche 15 bis 20, wobei der Hybridvektor einen Hefepromotor aufweist, der ausgewählt ist aus der Gruppe bestehend aus dem MFα1 Promotor; CYC1 Promotor; den galactoseinduzierbaren Promotoren GAL1, GAL7 und GAL10; den glycolytischen Enzympromotoren TPI und PGK; dem TRP1 Promotor, dem CUP1 Promotor; dem PHO5 Promotor, dem ADH1 Promotor und dem HSP Promotor.

## Revendications

1. Procédé de production d'un polypeptide à courte chaîne, à structure ouverte, hétérologue dans une levure, comportant (a) la culture d'une levure ayant une activité réduite de protéase Yap3, ladite levure étant transformée à l'aide d'un vecteur hybride comportant un promoteur de levure relié de manière opérationnelle à une séquence d'ADN codant pour le polypeptide hétérologue ayant de 10 à 70 acides aminés dans la chaîne polypeptidique, et ayant de 0 à 3 ponts disulfure dans la structure, et (b) l'isolation dudit polypeptide.

2. Procédé selon la revendication 1, dans lequel le polypeptide hétérologue a de 10 à 55 acides aminés dans la chaîne polypeptidique.

3. Procédé selon la revendication 1, dans lequel le polypeptide hétérologue a de 10 à 50 acides aminés dans la chaîne polypeptidique.

4. Procédé selon la revendication 1, dans lequel le polypeptide hétérologue a de 15 à 40 acides aminés dans la chaîne polypeptidique.

5. Procédé selon la revendication 1, dans lequel le polypeptide hétérologue a de 25 à 35 acides aminés dans la chaîne polypeptidique.

6. Procédé selon l'une quelconque des revendications 1 à 5, dans lequel le polypeptide hétérologue a un pont disulfure dans la structure.

7. Procédé selon l'une quelconque des revendications 1 à 6, dans lequel la levure présente un manque d'activité de protéase Yap3.

8. Procédé selon la revendication 1, dans lequel la levure est sélectionnée parmi le groupe constitué de Saccharomyces cerevisiae, Saccharomyces kluyveri, Schizosaccharomyces pombe, Kluyveromyces lactis, Hansenula polymorpha, Pichia pastoris, Pichia methanolica, Pichia kluyveri, Yarrowia lipolytica, Candida sp., Candida utilis, Candida cacaoi, Geotrichum sp., et Geotrichum fermentans.

9. Procédé selon la revendication 8, dans lequel la levure est S. cerevisiae.

10. Procédé selon l'une quelconque des revendications 1 à 9, dans lequel la levure a de plus une activité protéase réduite sélectionnée parmi le groupe de protéases codées par des gènes BAR1, STE13, PRA, PRB, KEX1, PRC, CPS, et des homologues de YAP3 MKC7, YAP3-link (codé par GenBank numéro d'accession X89514 : pos. 25352 à 26878), YIV9 (Swiss Prot numéro d'accession P40583), et de l'aspartyle protéase (IV) (codée par GenBank numéro d'accession U28372 : pos. 326 à 2116).

11. Procédé selon l'une quelconque des revendications 1 à 9, dans lequel la levure a de plus la mutation pep4-3.

12. Procédé selon la revendication 1, dans lequel le promoteur de levure est sélectionné parmi le groupe constitué du promoteur MFα1, du promoteur CYC1, des promoteurs inductibles par galactose GAL1, GAL7 et GAL10, des promoteurs d'enzyme glycolytique TPI et PGK, du promoteur TRP1, du promoteur CUP1, du promoteur PHO85, du promoteur ADH1, et du promoteur HSP.

13. Procédé selon l'une quelconque des revendications précédentes, dans lequel le vecteur hybride comporte une séquence d'ADN codant pour une séquence leader qui est une pré-séquence (signal) ou une pré-pro-séquence reliée dans le cadre de lecture correct à la séquence d'ADN codant pour le polypeptide hétérologue.

14. Procédé selon l'une quelconque des revendications précédentes, dans lequel le polypeptide hétérologue est sélectionné parmi le groupe constitué de GRPP (polypeptide apparenté à une glicentine), de glucagon, de GLP-1 (peptide de type glucagon 1), et de GLP-2 (peptide de type glucagon 2), de GLP-1(7-36), de GLP-1(7-35)-(R36A), de GLP-2-(F22Y), de GLP-2-(A19T+34Y), de GLP-2-(A2G) et de GLP-2-(A19T), et d'autres analogues ayants des changements, des ajouts et/ou des délétions de 1 à 3 acides aminés.

15. Culture de cellules de levure **caractérisée en ce qu'**elles ont une activité Yap3p réduite et qu'elles contiennent une première séquence d'ADN codant pour un polypeptide hétérologue ayant de 10 à 70 acides aminés et ayant de 0 à 3 ponts disulfure dans la structure, et une seconde séquence d'ADN codant pour une séquence leader amenant ledit polypeptide à être sécrété depuis la levure.

16. Culture selon la revendication 15, dans laquelle les cellules de levure présentent un manque d'activité Yap3p.

17. Culture selon l'une quelconque des revendications 15 ou 16, dans laquelle la levure est sélectionnée parmi le groupe constitué de Saccharomyces cerevisiae, Saccharomyces kluyveri, Schizosaccharomyces pombe, Kluyveromyces lactis, Hansenula polymorpha, Pichia pastoris, Pichia methanolica, Pichia kluyveri, Yarrowia lipolytica, Candida sp., Candida utilis, Candida cacaoi, Geotrichum sp., et Geotrichum fermentans.

18. Culture selon la revendication 17, dans laquelle la levure est S. cerevisiae.

19. Culture selon l'une quelconque des revendications 15 à 18, dans laquelle les cellules de levure ont de plus une activité protéase réduite sélectionnée parmi le groupe constitué de protéases codées par des gènes de BAR1, STE13, PRA, PRB, KEX1, PRC, CPS, et des homologues de YAP3 MKC7, YAP3-link (codé par GenBank numéro d'accession X89514 : pos. 25352 à 26878), YIV9 (Swiss Prot numéro d'accession P40583), et de l'aspartyle protéase (IV) (codée par GenBank numéro d'accession U28372 : pos. 326 à 2116).

20. Culture selon l'une quelconque des revendications 15 à 18, dans laquelle les cellules de levure ont de plus la mutation pep4-3.

21. Culture selon l'une quelconque des revendications 15 à 20, dans laquelle le vecteur hybride comporte un promoteur de levure sélectionné parmi le groupe constitué du promoteur MFα1, du promoteur CYC1, des promoteurs inductibles par galactose GAL1, GAL7 et GAL10, des promoteurs d'enzyme glycolytique TPI et PGK, du promoteur TRP1, du promoteur CUP1, du promoteur PHO85, du promoteur ADH1, et du promoteur HSP.
